(19) Europäisches Patentamt
European Patent Office
Office européen des brevets

(11) Publication number: 0 021 521
B1

(12) # EUROPEAN PATENT SPECIFICATION

(45) Date of publication of patent specification: 15.12.82

(51) Int. Cl.³: C 07 C 69/743, C 07 B 29/00 //A01N53/00

(21) Application number: 80200565.2

(22) Date of filing: 16.06.80

(54) **Process for the preparation of dihalovinyl compounds.**

(30) Priority: 03.07.79 GB 7923143

(43) Date of publication of application: 07.01.81 Bulletin 81/1

(45) Publication of the grant of the patent: 15.12.82 Bulletin 82/50

(84) Designated Contracting States: CH DE FR GB IT LI NL

(56) References cited:
COLLECTION OF CZECHOSLOVAK CHEMICAL COMMUNICATIONS, 7. XXVI. 1959 FARKAS et al. "Relation between chemical structure and insecticidal activity in pyrethroid compounds I. An analogue of chrysanthemic acid containing chlorine in the side chain", page 2230—2236.

(73) Proprietor: SHELL INTERNATIONALE RESEARCH MAATSCHAPPIJ B.V.
Carel van Bylandtlaan 30
NL-2596 HR Den Haag (NL)

(72) Inventor: Kramer, Petrus Anthonius
Badhuisweg 3
NL-1031 CM Amsterdam (NL)
Inventor: Verbrugge, Pieter Adriaan
Badhuisweg 3
NL-1031 CM Amsterdam (NL)

(74) Representative: Rogers, Roy Charles et al,
4 York Road
London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 021 521 B1

Courier Press, Leamington Spa, England.

# Process for the preparation of dihalovinyl compounds

This invention relates to a process for the production of dihalovinyl compounds. Some of these compounds may be employed as intermediates in the manufacture of pyrethroids which is an important group of new pesticides having outstanding insecticide properties coupled with a low mammalian toxicity.

Many pyrethroids contain a dihalovinyl group and thus the present invention is of considerable use in the manufacture of such pyrethroids.

According to Coll. Czech. Chem. Comm. *24* (1959) 2230—2236 3 - methyl - 1 - trichloromethyl - 2 - butenyl acetate and 3 - methyl - 1 - trichloromethyl - 3 - butenyl acetate can be converted with zinc and acetic acid into 1,1 - dichloro - 4 - methyl - 1,3 - pentadiene and 1,1 - dichloro - 4 - methyl - 1,4 - pentadiene, respectively.

Surprisingly it has now been found that certain esters afford dihalovinyl compounds by reaction with zinc in the absence of an alkanoic acid.

Accordingly, the invention provides a process for the preparation of a dihalovinyl compound of the general formula:

$$R^1-\underset{\underset{R^2}{|}}{C}=CHal_2, \qquad (I)$$

wherein $R^1$ represents an optionally-substituted cyclopropyl or an optionally-substituted alkyl group, $R^2$ a methyl group or a hydrogen atom or $R^1$ and $R^2$, together with the carbon atom to which they are attached jointly form a cycloalkylidene group, and each Hal stands for a chlorine or bromine atom, characterized in that a dihalophosphite of the general formula:

$$R^1-\overset{\overset{CHal_3}{|}}{\underset{\underset{R^2}{|}}{C}}-O-PQ_2, \qquad (II)$$

wherein $R^1$, $R^2$ and Hal have the same meaning as in formula I and Q represents a halogen atom, is contacted with zinc in the absence of an alkanoic acid with fewer than 5 carbon atoms per molecule.

The zinc is suitably employed as powdered zinc, for example zinc dust. The molar ratio of zinc to the dihalophosphite of formula II is not critical and may vary within a wide range, suitably between 1 : 1 and 10 : 1 and preferably between 1 : 1 and 5 : 1. The process is suitably carried out at a temperature in the range −20°C to 100°C, for example 0 to 50°C. Temperatures in the range 0 to 35°C, are usually very suitable.

Preferred dihalophosphites of formula II are those in which $R^1$ represents an optionally-substituted cyclopropyl group, particularly an optionally 3 - substituted 2,2 - dimethylcyclopropyl group and $R^2$ represents a hydrogen atom, because many of such compounds are intermediates in the preparation of pyrethroid insecticides. Among these compounds those in which $R^1$ represents a 2 - alkoxycarbonyl - 3,3 - dimethylcyclopropyl group, in which the alkoxy group has fewer than five carbon atoms, are preferred. This alkoxy group is preferably a methoxy or an ethoxy group; methoxy groups are most preferred. Other preferred dihalophosphites of formula II are those in which $R^1$ represents a 2 - (2,2 - dimethoxyethyl) - 3,3 - dimethylcyclopropyl group.

Good results have also been obtained with dihalophosphites of formula II in which $R^1$ represents an optionally-substituted alkyl group, particularly with fewer than ten carbon atoms. Examples of such alkyl groups are isopropyl, ethyl and methyl groups. Very suitable are dihalophosphites of formula II in which $R^1$ represents a cyclopropyl-substituted methyl group, particularly a 2,2-dimethyl-3-(2-oxypropyl)cyclopropylmethyl group.

Q in the group $-PQ_2$ of formula II preferably represents a chlorine or bromine atom. Preferred dihalophosphites of formula II are 2,2,2 - tribromo - 1 - (2 - ethoxycarbonyl - 3,3 - dimethylcyclopropyl)ethyl dichlorophosphite (compound 1) and 2,2,2 - trichloro - 1 - (2 - ethoxycarbonyl - 3,3 - dimethylcyclopropyl)ethyl dichlorophosphite (compound 2), as shown below.

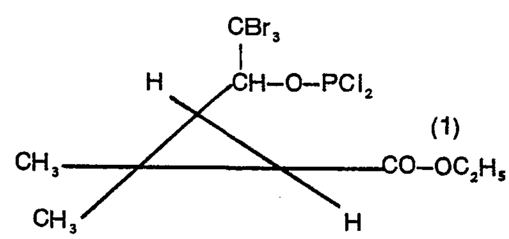

(1)

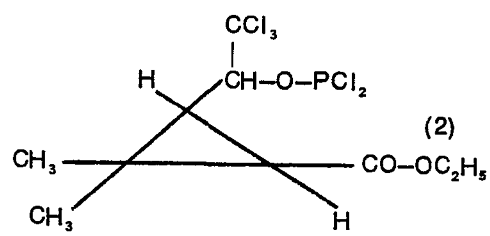

(2)

The dihalophosphites of formula II can be obtained by reacting an alcohol of the general formula:

$$R^1-\overset{\overset{CHal_3}{|}}{\underset{\underset{R^2}{|}}{C}}-O-H$$

wherein $R^1$, $R^2$ and Hal have the same meaning as in formula II, with a phosphorous trihalide. Alternatively they may be prepared by reacting a carbonate of the general formula:

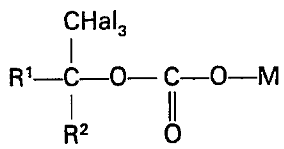
(III)

wherein $R^1$, $R^2$ and Hal have the same meaning as in formula I and M represents an alkali metal atom, with a phosphorus trihalide to give a compound of formula II.

The carbonates of formula III can be prepared by reacting a carbonyl compound of the general formula:

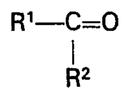
(IV)

wherein $R^1$ and $R^2$ have the same meaning as in formula III, with a trihaloacetate of the general formula:

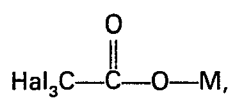
(V)

wherein M and Hal have the same meaning as in formula III, under substantially anhydrous conditions and in the presence of a highly polar, aprotic, inert solvent.

Suitable highly polar, aprotic, inert solvents include, N,N-dimethylformamide, N,N-dimethylacetamide, dimethyl sulphoxide, hexamethylphosphorictriamide —$[(CH_3)_2N]_3PO$—, tetrahydrothiophene 1,1-dioxide (also named "sulpholane"), 3-methyltetrahydrothiophene 1,1-dioxide, N-methyl-2-pyrolidone and acetonitrile. The carbonates of formula III and the dihalophosphites of formula II and the dihalovinyl compounds of formula I are preferably prepared in the same reaction vessel or pot or sequence of reaction vessels or pots.

Many common organic solvents possess some degree of polarity arising from asymmetry in their molecules, but an essential feature of the latter process is that the solvent should be "highly polar", a term used herein to denote the presence of a dielectric constant, measured at 25°C, of at least 25. The term "aprotic" as used herein denotes a solvent which is free from hydrogen atoms that are able to form hydrogen bonds with anions. These definitions are in accordance with "Physical Chemistry of Organic Solvent Systems", edited by A. K. Corrington and T. Dickinson, Plenum Press (1973), pages 332 and 333. Thus, alcohols and glycols do not come within this definition since the hydrogen atom of the hydroxyl group(s) form a hydrogen bond with the negative oxygen atom of the starting aldehyde. Benzene, carbon tetrachloride, 1,2-dimethoxyethane and pyridine also do not come within the definition since they are not highly polar. Nitromethane is highly polar and aprotic but not inert.

According to an aspect of the present invention, the dihalophosphite starting material of the general formula II can be prepared by a process which comprises the reaction of a carbonyl compound of the general formula:

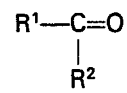
(IV)

wherein $R^1$ and $R^2$ have the same meaning as in formula I with a trihaloacetate of the general formula:

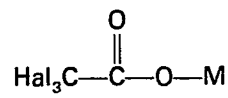
(V)

wherein Hal has the same meaning as in formula I and M represents an alkali metal atom, under substantially anhydrous conditions and in the presence of a highly polar, aprotic, inert solvent, with formation of a carbonate of the general formula:

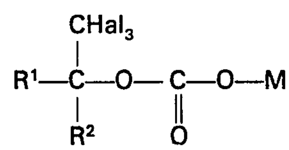
(III)

wherein $R^1$, $R^2$ and Hal have the same meaning as in formula I and M has the same meaning as in formula V, followed by reaction of the carbonate of formula III with a phosphorus trihalide to give a compound of formula II.

As mentioned above the route to the dihalovinyl compounds starting from the carbonyl compound of general formula IV has the advantage that it can be carried out in one reaction zone, under substantially similar reaction conditions, and without separating the intermediates of general formulae II and III.

The following examples further illustrate the invention. Yields and purities were determined by nuclear magnetic resonance (NMR) spectroscopy.

Example I

*Preparation of 1,1-dichloro-3-methyl-1-butene*

An NMR tube was charged with isobutyraldehyde (0.59 mmol) and a solution of sodium trichloroacetate (0.64 mmol) in N,N-dimethylformamide (0.4 ml). After standing for 30 min. at 20°C — the solution then contained sodium 1,1,1-trichloro-3-methyl-2-butyl carbonate — phosphorous trichloride (0.64 mmol) was added which caused an exothermic reaction with formation of 1,1,1-trichloro-3-methyl-2-butyl dichlorophosphite. The, zinc powder (0.96 mmol) was added and the mixture in the tube was

shaken at 20°C for 3 min. The title compound was obtained in a yield of 60%.

Example II

*Preparation of ethyl trans 2-(2,2-dibromovinyl)-3,3-dimethylcyclopropanecarboxylate*

A 25-ml flask provided with a magnetic stirrer and calcium chloride tube and placed in an ice bath was charged with a solution of ethyl trans 2 - (2,2,2 - tribromo - 1 - hydroxyethyl) - 3,3 - dimethylcyclopropanecarboxylate (1.18 mmol) in N,N-dimethylformamide (5 ml) and then with phosphorus trichloride (1.30 mmol). After stirring for one hour — the solution then contained trans 2,2,2 - tribromo - 1 - (2 - ethoxycarbonyl - 3,3 - dimethylcyclopropyl)ethyl dichlorophosphite — zinc powder (2.60 mmol) was added at 0°C over a period of 40 min. Then, the reaction mixture was poured out in water (50 ml) and the mixture obtained was three times extracted with n-pentane (10 ml portions). The combined extract phases were twice washed with water (5 ml portions), the washed liquid was dried over anhydrous magnesium sulphate and the solvent was evaporated from the dried liquid at sub-atmospheric pressure, leaving a colourless oil of which the content of the title compound was more than 90%. The conversion of the starting ethyl ester was more than 95% with a selectivity to the title compound of more than 65%.

The NMR spectrum of trans 2,2,2 - tribromo - 1 - (2 - ethoxycarbonyl - 3,3 - dimethylcyclopropyl)ethyl dichlorophosphite was recorded at 90 MHz and showed the following absorptions relative to a tetramethylsilane standard.

$\delta$=1.24 ppm, singlet, C*$H_3$*—C—$CH_3$

$\delta$=1.24 ppm, triplet, C(O)O—$CH_2$—C*$H_3$*

$\delta$=2.08 ppm, doublet, O=C—C*H*

$\delta$=4.14 ppm, multiplet, $CH_3$—C*$H_2$*

$\delta$=1.38 ppm, singlet, $CH_3$—C—$CH_3$

$\delta$=1.93 ppm, double doublet, $CBr_3$—C—C*H*

J=5Hz (with the ring proton) and J=9Hz (with the side chain proton)

$\delta$=4.50 ppm, double doublet, $CBr_3$—C*H*; $J_{PH}$ = 11 Hz

## Claims

1. Process for the preparation of a dihalovinyl compound of the general formula:

$R^1$—C=$CHal_2$,
|
$R^2$ (I)

wherein $R^1$ represents an optionally-substituted cyclopropyl or an optionally-substituted alkyl group, $R^2$ a methyl group or a hydrogen atom or $R^1$ and $R^2$, together with the carbon atom to which they are attached jointly form a cycloalkylidene group, and each Hal stands for a chlorine or bromine atom, characterized in that a dihalophosphite of the general formula:

$CHal_3$
|
$R^1$—C—O—$PQ_2$,
|
$R^2$ (II)

wherein $R^1$, $R^2$ and Hal have the same meaning as in formula I and Q represents a halogen atom, is contacted with zinc in the absence of an alkanoic acid with fewer than 5 carbon atoms per molecule.

2. A process according to claim 1, characterized in that $R^1$ represents an optionally-substituted cyclopropyl group and $R^2$ is a hydrogen atom.

3. A process according to claim 2, characterized in that $R^1$ represents a 2-alkoxycarbonyl-3,3-dimethylcyclopropyl group in which the alkoxy group has fewer than five carbon atoms.

4. A process as claimed in claim 2 or 3, characterized in that $R^1$ represents a 2-(2,2-dimethoxyethyl)-3,3-dimethylcyclopropyl group.

5. A process according to claim 1, characterized in that $R^1$ represents an optionally-substituted alkyl group with fewer than ten carbon atoms.

6. A process according to any one of the preceding claims, characterized in that each of the two Q atoms in the group —$PQ_2$ is a chlorine or bromine atom.

7. A process according to any one of the preceding claims, characterized in that the zinc is powdered zinc.

8. A process according to any one of the preceding claims, characterized in that the molar ratio of zinc to dihalophosphite of formula II is in the range 1:1 to 5:1.

9. A process according to any one of the preceding claims, characterized in that it is carried out at a temperature in the range of from −20°C to 100°C.

10. A process according to any one of the preceding claims, characterized in that the

starting dihalophosphite of the general formula II is prepared by reaction of a carbonyl compound of the general formula:

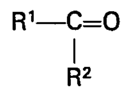
(IV)

wherein $R^1$ and $R^2$ have the same meaning as in formula I with a trihaloacetate of the general formula:

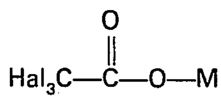
(V)

wherein Hal has the same meaning as in formula I and M represents an alkali metal atom, under substantially anhydrous conditions and in the presence of a highly polar, aprotic, inert solvent, with formation of a carbonate of the general formula:

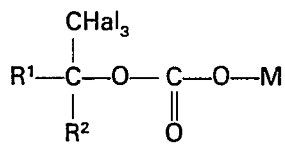
(III)

wherein $R^1$, $R^2$ and Hal have the same meaning as in formula I and M has the same meaning as in formula V, followed by reaction of the carbonate of formula III with a phosphorus trihalide to give a compound of formula II.

**Revendications**

1. Procédé pour la préparation d'un composé dihalovinyl de la formule générale:

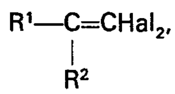
(I)

dans laquelle $R^1$ représente un groupe cyclopropyle éventuellement substitué ou un groupe alcoyle éventuellement substitué, $R^2$ représente un groupe méthyle ou un atome d'hydrogène ou $R^1$ et $R^2$, en même temps que l'atome de carbone sur lequel ils sont fixés, forment ensemble un groupe cycloalcoylidène, et chaque Hal représente un atome de chlore ou de brome, caractérisé en ce qu'un dihalophosphite de la formule générale:

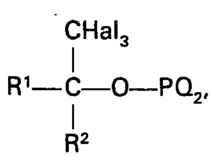
(II)

dans laquelle $R^1$, $R^2$ et Hal ont la même signification que dans la formule I et Q représente un atome d'halogène, est mis en contact avec du zinc en l'absence d'un acide alcanoïque ayant moins de 5 atomes de carbone par molécule.

2. Procédé selon la revendication 1, caractérisé en ce que $R^1$ représente un groupe cyclopropyle éventuellement substitué et $R^2$ est un atome d'hydrogène.

3. Procédé selon la revendication 2, caractérisé en ce que $R^1$ représente un groupe 2-alkoxycarbonyl-3,3-diméthylcyclopropyle dans lequel le groupe alcoxy a moins de cinq atomes de carbone.

4. Procédé selon la revendication 2 ou 3, caractérisé en ce que $R^1$ représente un groupe 2 - (2,2 - diméthoxyéthyl) - 3,3 - diméthylcyclo - propyle.

5. Procédé selon la revendication 1, caractérisé en ce que $R^1$ représente un groupe alcoyle éventuellement substitué ayant moins de dix atomes de carbone.

6. Procédé selon l'une quelconque des revendications précédentes, caractérisé en ce que chacun des deux atomes Q dans le groupe $—PQ_2$ est un atome de chlore ou de brome.

7. Procédé selon l'une quelconque des revendications précédentes, caractérisé en ce que le zinc est du zinc pulvérisé.

8. Procédé selon l'une quelconque des revendications précédentes, caractérisé en ce que le rapport molaire du zinc au dihalophosphite de formule II est compris entre 1:1 et 5:1.

9. Procédé selon l'une quelconque des revendications précédentes, caractérisé en ce qu'il est mis en oeuvre à une température comprise entre –20°C et 100°C.

10. Procédé selon l'une quelconque des revendications précédentes, caractérisé en ce que le dihalophosphite de départ de la formule générale II est préparé par réaction d'un composé carbonylé de la formule générale:

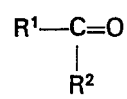
(IV)

dans laquelle $R^1$ et $R^2$ ont la même signification que dans la formule I avec un trihaloacétate de la formule générale:

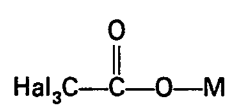
(V)

dans laquelle Hal a la même signification que dans la formule I et M représente un atome de métal alcalin, dans des conditions sensiblement anhydres et en présence d'un solvant inerte, aprotique, hautement polaire, avec formation d'un carbonate de la formule générale:

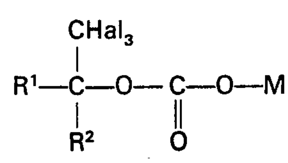
(III)

dans laquelle $R^1$, $R^2$ et Hal ont la même signification que dans la formule I et M a la même signification que dans la formule V, suivie de la réaction du carbonate de formule III avec un trihalogénure de phosphore pour donner un composé de formule II.

**Patentansprüche**

1. Verfahren zur Herstellung einer Dihalogenvinylverbindung der allgemeinen Formel

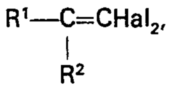

(I)

in der $R^1$ eine gegebenenfalls substituierte Cyclopropyloder eine gegebenenfalls substituierte Alkylgruppe, $R^2$ eine Methylgruppe oder ein Wasserstoffatom oder, $R^1$ und $R^2$ zusammen mit dem Kohlenstoffatom, an das sie gebunden sind, eine Cycloalkylidengruppe bedeuten und jedes Hal für ein Chlor- oder Bromatom steht, dadurch gekennzeichnet, daß ein Dihalogenphosphit der allgemeinen Formel

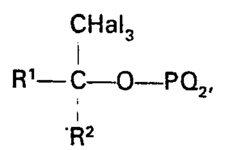

(II)

in der $R^1$, $R^2$ und Hal die in Formel (I) angegebene Bedeutung haben und Q ein Halogenatom bedeutet, zusammengebracht wird mit Zink in Abwesenheit einer Alkansäure mit weniger als 5 Kohlenstoffatomen pro Molekül.

2. Verfahren nach Anspruch 1, dadurch gekennzeichnet, daß $R^1$ eine gegebenenfalls substituierte Cyclopropylgruppe und $R^2$ ein Wasserstoffatom bedeuten.

3. Verfahren nach Anspruch 2, dadurch gekennzeichnet, daß $R^1$ eine 2-Alkoxycarbonyl-3,3-dimethylcyclopropylgruppe bedeutet, bei der die Alkoxygruppe weniger als 5 Kohlenstoffatome besitzt.

4. Verfahren nach Anspruch 2 oder 3, dadurch gekennzeichnet, daß $R^1$ eine 2 - (2,2 - Dimethoxyethyl) - 3,3 - dimethylcyclopropyl - gruppe bedeutet.

5. Verfahren nach Anspruch 1, dadurch gekennzeichnet, daß $R^1$ eine gegebenenfalls substituierte Alkylgruppe mit weniger als 10 Kohlenstoffatomen bedeutet.

6. Verfahren nach einem der vorhergehenden Ansprüche, dadurch gekennzeichnet, daß jedes der beiden Q-Atome in der Gruppe $—PQ_2$ ein Chlor- oder Bromatom ist.

7. Verfahren nach einem der vorhergehenden Ansprüche, dadurch gekennzeichnet, daß das Zink pulverförmiges Zink ist.

8. Verfahren nach einem der vorhergehenden Ansprüche, dadurch gekennzeichnet, daß das Molverhältnis von Zink zu Dihalogenphosphit der Formel (II) im Bereich von 1:1 bis 5:1 liegt.

9. Verfahren nach einem der vorhergehenden Ansprüche, dadurch gekennzeichnet, daß es bei einer Temperatur im Bereich von —20 bis 100°C durchgeführt wird.

10. Verfahren nach einem der vorhergehenden Ansprüche, dadurch gekennzeichnet, daß Ausgangs-Dihalogenphosphit der allgemeinen Formel (II) hergestellt wird durch Umsetzung einer Carbonylverbindung der allgemeinen Formel

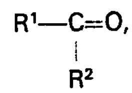

(IV)

in der $R^1$ und $R^2$ die in Formel (I) angegebene Bedeutung haben, mit einem Trihologenacetat der allgemeinen Formel

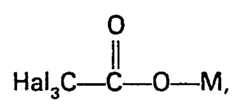

(V)

in der Hal die für Formel (I) angegebene Bedeutung hat und M ein Alkalimetallatom bedeutet, unter im wesentlichen wasserfreien Bedingungen und in Gegenwart eines hoch polaren, aprotischen, inerten Lösungsmittels unter Bildung eines Carbonats der allgemeinen Formel

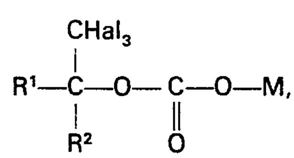

(III)

in der $R^1$, $R^2$ und Hal die für Formel (I) angegebene Bedeutung haben und M die für Formel (V) angegebene Bedeutung hat, und anschließende Reaktion des Carbonats der Formel (III) mit einem Phosphortrihalogenid unter Bildung einer Verbindung der Formel (II).